# EUROPEAN PATENT APPLICATION

(11) **EP 4 008 335 A1**
(43) Date of publication of application: **08.06.2022**
(21) Application number: 20212283.4
(22) Date of filing: 07.12.2020
(51) Int. Cl.: A61K 36/61, A61P 17/00, A61K 8/67, A61K 31/045, A61K 31/05, A61K 31/197, A61K 36/53, A61K 36/752, A61K 47/10, A61L 2/00, A61P 31/10

(54) **COMPOSITION COMPRISING MELALEUCA ALTERNIFOLIA ESSENTIAL OIL, USE AND METHOD**

(71) Applicant: Vingerhoets Jensen, Susanne, 5049 AD Tilburg (NL)
(72) Inventor: Vingerhoets Jensen, Susanne, 5049 AD Tilburg (NL)
(74) Representative: Arnold & Siedsma

(57) **Abstract**

A composition is provided, comprising:
a. Melaleuca Alternifolia essential oil,
b. from 0 to 99% by weight ethanol relative to the total composition,
c. from 0 to 10% by weight of a benzoate compound relative to the total composition,
d. from 0.0005 to 10% by weight Linalool relative to the total composition,
wherein the total percentage of the benzoate compound and Linalool together amounts to at least 0.1% by weight of the weight of the total composition.

Furthermore, an antifungal agent comprising Melaleuca Alternifolia essential oil is provided, a composition according to the invention for use in the prevention and/ or treatment of fungal infection, and a method for preventing and/or treating fungal infection on a skin surface.

## Description

### Field of Invention

The field of the invention relates to a composition comprising Melaleuca Alternifolia essential oil. The field of the invention further relates to an antifungal agent comprising Melaleuca Alternifolia essential oil, a composition according to the invention for use in the prevention and/ or treatment of fungal infection, and method for preventing and/or treating fungal infection on a skin surface. The field of invention further relates to a packaging comprising said composition.

### Background

Many people suffer from fungal infections of the skin. Feet in particular are often infected with a fungus. One of the best known fungal foot infections is tinea pedis, also known as 'athlete's foot'. Tinea pedis usually occurs in the vicinity of toes, and can have a very adverse effect on the nails of the toes. Several remedies for this disorder are known which can be applied to the infected body parts, including clotrimazole, miconazole, terbinafine, ciclopirox and sulconazole. Remedies for internal use are also known, including terbinafine and itraconazole. The drawback of treatment with these known remedies is that treatments have not been found to be very successful. Fungi are not found to disappear, even after prolonged treatment, or the infection returns after a period of time. Furthermore, side-effects can occur for some of the known remedies.
The use of Melaleuca Alternifolia essential oil for treating fungal infections of the skin is also known. However, Melaleuca Alternifolia essential oil may have the drawback that, when applied in an excessive amount on the skin, it may induce irritations of the skin.

The object of the invention is to provide an improved antifungal agent, which provides effective antifungal properties while reducing or preventing dermatological side-effects, such as irritation reactions of the skin.

### Summary

According to a first aspect of the invention there is provided a composition, comprising:
a. Melaleuca Alternifolia essential oil,
b. from 0 to 99% by weight ethanol relative to the total composition,
c. from 0 to 10% by weight of a benzoate compound relative to the total composition,
d. from 0.0005 to 10% by weight Linalool relative to the total composition,
wherein the total percentage of the benzoate compound and Linalool together amounts to at least 0.1% by weight of the weight of the total composition.

According to another aspect of the invention there is provided a packaging comprising a composition according to the invention, preferably wherein the packaging also comprises an applicator adapted to apply the composition to a surface.

According to another aspect of the invention there is provided an antifungal agent obtainable by the method of:
a. providing Melaleuca Alternifolia essential oil;
b. providing Linalool;
c. providing benzoate compound;
d. providing ethanol and water;
e. mixing Melaleuca Alternifolia essential oil in any order with ethanol and water, said benzoate compound and said linalool.

According to another aspect of the invention there is provided a composition according to the invention or the antifungal agent according to the invention for use in the prevention and/ or treatment of fungal infection on a skin surface, such as a foot, of a patient or for use in the prevention and/ or treatment of fungal infection on a skin surface, such as a foot, of an animal.

According to another aspect of the invention there is provided a method for preventing and/or treating fungal infection on a skin surface, in particular a foot, of a patient or of an animal, comprising administering the composition according to the invention or the antifungal agent according to the invention.

It has been found that combining one or more, preferably combining all, of Melaleuca Alternifolia essential oil with benzoic acid compound and Linalool and Ethanol enhances the antifugal properties. In addition a composition may be derived from a combination of these components, which prevents or treats fungal infection on a skin surface while preventing dermatological side-effects of long term use, such as irritation reactions of the skin.

### (Melaleuca Alternifolia essential oil)

Melaleuca Alternifolia essential oil is an etheric oil obtained from the tea tree, also known as Manuka. Although types of tea tree other than Melaleuca Alternifolia are known, Melaleuca Alternifolia is the most readily available. The essential oil is, in contrast to many other etheric oils, not irritating to the skin. Typical chemical compounds found in Melaleuca Alternifolia essential oil are terpinene-4-ol and cineole.

In an embodiment, the composition comprises at least 1% by weight of Melaleuca Alternifolia essential oil, and preferably at most 10 % by weight, more preferably at most 5 % by weight, of Melaleuca Alternifolia essential oil.

### (Benzoate compounds)

The best known benzoate compounds are benzoic acid and sodium benzoate, although other salts of benzoic acid can also be used. The stated percentage by weight assumes sodium benzoate, though it will be apparent that when other benzoate salts are used the percentages must be modified to the relevant molecular weight. A choice is preferably made for a benzoate with a good solubility in a determined composition, wherein sodium benzoate is particularly suitable for polar compositions (for instance an aqueous composition), while benzoic acid is particularly suitable for apolar compositions (for instance an oleaginous composition).

### (Linalool)

Linalool refers to two enantiomers of a naturally occurring terpene alcohol. It may be found in flowers and spice plants. Linalool has the chemical formula: 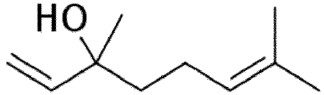 Linalool has a stereogenic center at C₃ and therefore there are two stereoisomers: (R)-(-)-linalool is also known as licareol and (S)-(+)-linalool is also known as coriandrol.

Preferably, the amount of Linalool is from 0.0005 to 1% by weight Linalool relative to the total composition.

### (D-limonene)

Limonene is a colorless liquid aliphatic hydrocarbon classified as a cyclic monoterpene, and is the major component in the oil of citrus fruit peels. Limonene has the chemical formula: 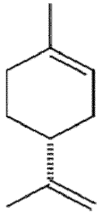 The D-isomer occurs in nature as the fragrance of oranges.

In an embodiment, the composition further comprises:
e. from 0.01 to 10% by weight D-Limonene relative to the total composition.
Preferably, the amount of D-Limonene is from 0.05 to 1% by weight D-Limonene relative to the total composition.

### (Carvacrol)

Carvacrol, or cymophenol, C₆H₃(CH₃)(OH)C₃H₇, is a monoterpenoid phenol. It has the chemical formula:

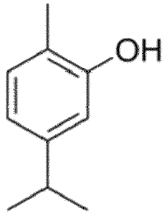

Cavacrol may be synthesized or may be obtained from a natural source.

### (Thymol)

Thymol (also known as 2-isopropyl-5-methylphenol, IPMP) is a natural monoterpenoid phenol derivative of p-Cymene, C₁₀H₁₄O. It is an isomeric of carvacrol. It has the chemical formula:

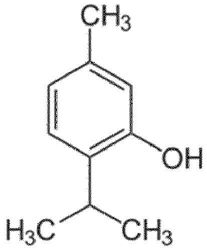

Thymol may be found in oil of thyme, and extracted from Thymus vulgaris (common thyme), and various other kinds of plants as a white crystalline substance of a pleasant aromatic odor. Thymol also provides the distinctive, strong flavor of the culinary herb thyme, also produced from T. vulgaris.

In an embodiment, the composition further comprises:
f. from 0 to 10% by weight Carvacrol relative to the total composition;
g. from 0 to 10% by weight Thymol relative to the total composition; and
wherein the total percentage of the Carvacrol, and Thymol together amounts to at least 0.01% by weight of the weight of the total composition.

In a particular embodiment, the amount of Carvacrol is from 0.05 to 1% by weight Carvacrol relative to the total composition. In a particular embodiment, the amount of Thymol is from 0.05 to 1% by weight Thymol relative to the total composition.

### (Diluent)

In an embodiment, the composition also comprises a dermatologically acceptable diluent, preferably wherein the diluent comprises water.

It is advantageous if the composition also comprises a dermatologically acceptable diluent. A diluent dilutes the active components (Melaleuca Alternifolia essential oil in combination with the benzoate compound and/or Linalool), so that an optimal distribution is achieved during application to a surface for treating. Ideally the active components are mixed substantially homogeneously with the rest of the composition. Different solid, liquid and/or gaseous substances or compositions can be used as diluents. The diluent can for instance be a solvent in which the active components dissolve. Liquid diluents which can be used are for instance water, aliphatic alcohols and other organic solvents. The composition can also be an emulsion, such as a lotion, ointment or cream. In a particular preferred embodiment the diluent is a gas, wherein the composition is formed into a foam. Another option is a solid substance, wherein the resulting composition can for instance be processed into a powder. In order to obtain the above stated compositions use generally has to be made of suitable excipients known in the cosmetic and pharmaceutical industry, such as surfactants, fillers and emulsifiers, which enhance mixing of the active components with the diluents and bring about the desired structure of the composition.

It is advantageous if the diluent is substantially a volatile solvent. A volatile solvent makes it possible to apply the active components in diluted distribution over a large surface, wherein after evaporation of the volatile solvent the active components remain behind in more concentrated form. A considerable antifungal effect is thus achieved. Volatile solvents are understood to mean liquids with a lower heat of evaporation than water (2.26 x 10⁶ J/kg). Examples are acetone, methyl ethyl ketone, water, ethanol and mixtures thereof. The volatile solvent preferably comprises at least 50% by weight of the composition.

### (Ethanol)

The composition according to the invention comprises ethanol. Ethanol has the particular advantage that when it evaporates it also extracts the water from the treated surface, thereby improving the antifungal effect. In addition, ethanol has a good capacity for dissolving the active components, whereby a homogeneous composition can be more readily obtained.

In addition, it has been found that a concentration of ethanol in the composition of at least 50% by weight or more enhances an antifungal effect of one or more of the components Melaleuca Alternifolia essential oil, benzoate compound and/or Linalool.

In particular, it has been found that a composition comprising at least 50% by weight of ethanol in addition to water increases the pH of the composition to above pH 8. The pH of the composition of higher than 8 enhances the antifugal effect of one or more of the components Melaleuca Alternifolia essential oil, benzoate compound and/or Linalool.

In addition, it has been found that another effect of the composition comprising at least 50% by weight of ethanol is that the skin and/or the nails are induced to accept the active components Melaleuca Alternifolia essential oil, benzoate compound and/or Linalool. Thereby, the antifugal effect of the one or more of the components Melaleuca Alternifolia essential oil, benzoate compound and/or Linalool is enhanced.

### (Panthenol)

In an embodiment, the composition comprises panthenol. In a particular embodiment, the composition comprises at least 0.5% by weight of panthenol relative to the total composition. Panthenol is the alcohol analog of pantothenic acid (vitamin B5), and is thus a provitamin of B5. In a particular example, the composition comprises D-panthenol. Panthenol has the chemical formula:

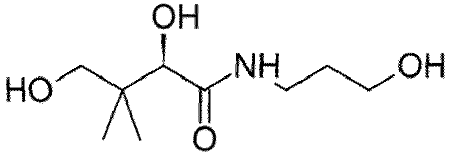

It has been found that panthenol supports the topological use of the antifungal composition. The skin and the nails are hydrated. The nails are strengthened, while the fungal infection is reduced.

### (Further combinations)

In an embodiment, the composition comprises at least 50% by weight, preferably at least 70% by weight, of ethanol and at least 0.5% by weight benzoate compound, relative to the total composition.

In an embodiment, the composition comprises at least 0.5% by weight of the benzoate compound, and at least 0.001% by weight of Linalool, relative to the total composition.
In a particular embodiment, the composition comprises at least 0.5% by weight of the benzoate compound, at least 0.001% by weight of Linalool and at least 50% by weight of ethanol, relative to the total composition.

In an embodiment, the composition comprises:
a. at least 0.05% by weight of Carvacrol; and / or
b. at least 0.05% by weight of Thymol.
In a particular embodiment, the composition comprises at least 0.5% by weight of the benzoate compound, at least 0.05% by weight of Carvacrol and at least 50% by weight of ethanol, relative to the total composition.
In a particular embodiment, the composition comprises at least 0.5% by weight of the benzoate compound, at least 0.05% by weight of Thymol and at least 50% by weight of ethanol, relative to the total composition.

### (Other components)

In a preferred embodiment the composition also comprises an aromatic substance. Melaleuca Alternifolia essential oil has an odour which is perceived by some as unpleasant, and this can be masked by an active quantity of aromatic substance. Diverse aromatic substances which can be used are commercially available, such as cinnamon, eucalyptus and menthol. Particularly suitable for this purpose are fruit aromas which have the advantage that only a relatively small quantity hereof is necessary to mask the odour of tea tree oil. Examples of suitable aromas are lemon and lime.

### (Denatonium benzoate)

In an embodiment, the composition comprises Denatonium benzoate and at least 0.5% by weight of a benzoate compound other than Denatonium benzoate, such as sodium benzoate, relative to the total composition.

Denatonium benzoate is a very bitter chemical compound, with bitterness thresholds of 0.05 ppm for the benzoate. The Denatonium benzoate may be used together with a sodium benzoate in the composition without reducing the antifugal properties of the composition. The Denatonium benzoate provides the benefit that the composition it signals clearly that the composition is not usable for oral administration, which prohibits a user to mistakenly drink or administer the composition orally. Denatonium benzoate is commercially available under the tradename Bitrex and Byte-X.

### (Packaging)

The invention also comprises a packaging comprising a composition according to the invention. For liquid compositions it is possible to envisage a bottle or other reclosable packaging. The packaging is preferably aroma-tight. The packaging more preferably comprises a predetermined quantity for treatment of a surface. The quantity can for instance be apportioned for the treatment of two feet. A separate package is thus used for each treatment, which improves hygiene. A plastic disposable package can be envisaged here, for instance a closed breakable bag having therein a powder or application fluid.

It is advantageous if the packaging also comprises an applicator adapted to apply the composition to a surface. An applicator can be envisaged here which is connected to a container for the composition, for instance a spray head for distributing a liquid composition according to the invention over a surface. A brush or sponge for applying the liquid can for instance also be envisaged. For the treatment of larger surfaces, for instance the floor of a sports hall or swimming pool, larger applicators can be envisaged such as a floor cloth for liquid compositions according to the invention.

### (Applying the composition)

The invention moreover comprises a method comprising of applying a composition according to the invention to a surface. Such a surface is thus protected in preventive manner from fungal infections. Existing fungal infections are also combatted by applying the composition according to the invention to a surface on a regular basis (preferably daily). The method can for instance be applied to surfaces in bathrooms, swimming pools and sports accommodation. The surface can also be a skin surface, in particular a foot, more particularly a toenail. When used on toenails, it is recommended to first remove as much as possible of the infected parts of the toenail before applying the composition. This results in a shorter period of treatment.

### (Experimental Section)

The invention will now be elucidated on the basis of the following examples.

The compositions A, B, C, D, E, F, G, H shown in table I were prepared by mixing the stated components by means of known methods. Formulations A-H are liquid formulations, although formulations in powder form can also be envisaged.

### Compositions A - F are according to the invention.

Formulation A is a formulation of tea tree oil (Melaleuca Alternifolia essential oil) with sodium benzoate and ethanol. Formulation B is a formulation of Melaleuca Alternifolia essential oil with linalool and ethanol. Formulation C comprises Melaleuca Alternifolia essential oil in combination with sodium benzoate, linalool and ethanol. Formulation D comprises Melaleuca Alternifolia essential oil in combination with sodium benzoate, Carvacrol and ethanol. Formulation E comprises Melaleuca Alternifolia essential oil in combination with sodium benzoate, thymol and ethanol. Formulation F comprises Melaleuca Alternifolia essential oil in combination with sodium benzoate, D-limonene and ethanol.

### Comparison formulations (not according to the invention)

Formulation G comprises Melaleuca Alternifolia essential oil in combination with sodium benzoate dissolved in the Melaleuca Alternifolia essential oil. Formulation G is an evaporable solution of Melaleuca Alternifolia essential oil with sodium benzoate dissolved in Melaleuca Alternifolia essential oil and ethanol, wherein the composition comprises 15% by weight of Melaleuca Alternifolia essential oil.

**Table I: Antifungal Compositions**

| Component | Percent by weight | | | | | | | |
|---|---|---|---|---|---|---|---|---|
| Formulation | A | B | C | D | E | F | G (comp) | H (comp) |
| Glycerol | - | - | - | - | - | - | 20 | 20 |
| Tea Tree Oil | 3 | 3 | 3 | 3 | 3 | 3 | 30 | 15 |
| PEG-40 Hydrogenated Castor | 2 | - | 2 | 2 | 2 | 2 | 2 | - |
| Sodium Benzoate | 0.59 | - | 0.59 | 0.59 | 0.59 | 0.59 | 0.59 | 0.59 |
| Linalool | - | 0.005 | 0.005 | - | - | - | - | - |
| D-Limonene | - | - | - | - | - | 0.1 | - | - |
| Carvacrol | - | - | - | 0.2 | - | - | - | - |
| Thymol | - | - | - | - | 0.2 | - | - | - |
| Panthenol | 1 | 1 | 1 | 1 | 1 | 1 | - | - |
| Ethanol | 70 | 70 | 70 | 70 | 70 | 70 | - | Rest |
| Water | Rest | Rest | Rest | Rest | Rest | Rest | Rest | - |

Excipients are also used in addition to the active components (Melaleuca Alternifolia essential oil, benzoate and ethanol). These excipients can if desired be replaced by other known substances with similar properties. Glycerol assists dissolving and homogenizing. PEG-40 Hydrogenated Castor oil is an emulsifier and surfactant which assists good mixing of the active components. Panthenol is an active additive which softens the skin and nurtures the nails. The pH of the compositions A - F having 70% by weight ethanol and about 10 - 25% by weight water is in the range of 8.5 - 9.5.

The different formulations were applied in a comparative test. A first test consisted of qualitative determination of the preventive antifungal effect. It was determined here to what extent the formulation was able to prevent fungal growth on a previously clean surface. The therapeutic effect of the compositions was further tested. Diverse people with foot fungus were herein treated daily with the composition for a period of six months. This is the period of time a nail needs on average to renew itself through growth. A usable antifungal agent stops the growth of the fungus such that the fungus no longer occurs in the renewed nail after six months.
In case during the therapeutic treatment any adverse effects were seen, this is noted in the remarks.

**Table II: Antifungal effects of the components based on the compositions A - H**

| **Ingredient** | **Preventive Antifungal effect** | **Therapeutic Antifungal effect** | **Remarks** |
|---|---|---|---|
| Tea tree oil | - | - | |
| Sodium benzoate | + | + | |
| Linalool | + | + | |
| Ethanol | + / - | + / - | |
| Tea tree oil + benzoate + Ethanol (A) | ++ | ++ | composition H contains too much Tea tree oil |
| Tea tree oil + Linalool + Ethanol (B) | ++ | ++ | |
| Tea tree oil + benzoate + Linalool + Ethanol (C) | ++++ | ++++ | |
| Tea tree oil + benzoate + Carvacrol + Ethanol (D) | +++ | +++ | |
| Tea tree oil + benzoate + Thymol + Ethanol (E) | +++ | +++ | |
| Tea tree oil + benzoate + D-Limonene + Ethanol (F) | +++ | +++ | |

Table II shows the results of the different types of composition for both preventive and therapeutic effect. The assessments are represented qualitatively, with the following meaning: (-: unsatisfactory/no antifungal effect, + some antifungal effect, ++ satisfactory antifungal effect, +++ strong antifungal effect, +++ very strong antifungal effect). The differences between satisfactory, strong, and very strong antifungal effect are determined by the quantity of formulation necessary to achieve the desired result, wherein less of the strong formulations or of the very strong formulations is required to arrive at a comparable result. A lower quantity of the formulations may be preferred to be used as it may reduce any risks on side effects.

The composition H compared to composition A showed that an amount of Tea tree oil of 15% by weight has the disadvantage that side effects of the Tea tree oil, such as irritation of the skin, can be observed, when long term using the composition H.
The composition G compared to composition A showed that a composition having Tea tree oil and sodium benzoate in water without using ethanol does not provide the synergistic effect of combining ethanol, in particular at least 50% by weight of ethanol, with the other components.

The above examples are non-limitative, and many variants of the composition according to the invention can be envisaged by a skilled person in the field. In particular, the ingredients which are not immediately significant for the antifungal effect can be replaced by other constituents known in dermatology,

## Claims

1. Composition, comprising:
a. Melaleuca Alternifolia essential oil,
b. from 0 to 99% by weight ethanol relative to the total composition,
c. from 0 to 10% by weight of a benzoate compound relative to the total composition,
d. from 0.0005 to 10% by weight Linalool relative to the total composition,
wherein the total percentage of the benzoate compound and Linalool together amounts to at least 0.1% by weight of the weight of the total composition.

2. Composition of claim 1, wherein the composition further comprises:
e. from 0.01 to 10% by weight D-Limonene relative to the total composition.

3. Composition of any one of the claims 1 - 2, wherein the composition further comprises:
f. from 0 to 10% by weight Carvacrol relative to the total composition;
g. from 0 to 10% by weight Thymol relative to the total composition; and
wherein the total percentage of the Carvacrol, and Thymol together amounts to at least 0.01% by weight of the weight of the total composition.

4. Composition of any one of the claims 1 - 3, wherein the composition comprises at least 1% by weight of Melaleuca Alternifolia essential oil, and preferably at most 10 % by weight, more preferably at most 5 % by weight, of Melaleuca Alternifolia essential oil.

5. Composition according to any one of the preceding claims, wherein the composition comprises at least 50% by weight, preferably at least 70% by weight, of ethanol and at least 0.5% by weight benzoate compound.

6. Composition according to any one of the preceding claims, wherein the composition comprises at least 0.5% by weight of the benzoate compound, and at least 0.001% by weight of Linalool.

7. Composition according to any one of the preceding claims, wherein the composition comprises:
a. at least 0.05% by weight of Carvacrol; and / or
b. at least 0.05% by weight of Thymol.

8. Composition according to any one of the preceding claims, wherein the composition comprises Denatonium benzoate and at least 0.5% by weight of a benzoate compound other than Denatonium benzoate, such as sodium benzoate.

9. Composition according to any one of the preceding claims, wherein the composition comprises panthenol.

10. Composition according to any one of the preceding claims, wherein the composition further comprises a dermatologically acceptable diluent, preferably wherein the diluent comprises water.

11. Packaging comprising a composition as claimed in any of the preceding claims 1-10, preferably wherein the packaging also comprises an applicator adapted to apply the composition to a surface.

12. Antifungal agent obtainable by the method of:
a. providing Melaleuca Alternifolia essential oil;
b. providing Linalool;
c. providing benzoate compound;
d. providing ethanol and water;
e. mixing Melaleuca Alternifolia essential oil in any order with ethanol and water, said benzoate compound and said linalool.

13. Composition according to any one of the claims 1 - 10 or the antifungal agent according to claim 12 for use in the prevention and/ or treatment of fungal infection on a skin surface, such as a foot, of a patient or for use in the prevention and/ or treatment of fungal infection on a skin surface, such as a foot, of an animal.

14. Method for preventing and/or treating fungal infection on a skin surface, in particular a foot, of a patient or of an animal, comprising administering the composition according to any one of the claims 1 - 10 or the antifungal agent according to claim 12.
